# EUROPÄISCHE PATENTSCHRIFT

(11) **EP 1 377 208 B1**
(45) Veröffentlichungstag und Bekanntmachung des Hinweises auf die Patenterteilung: **18.02.2009**
(21) Anmeldenummer: 02727299.6
(22) Anmeldetag: 04.04.2002
(51) Int. Cl.: A61B 5/04, A61B 5/0428

(54) **ERFASSUNG VON SIGNALEN BIOLOGISCHEN URSPRUNGS**
ARRANGEMENT AND METHOD FOR RECORDING SIGNALS OF BIOLOGICAL ORIGIN
ENSEMBLE ET PROCEDE D'ACQUISITION DE SIGNAUX D'ORIGINE BIOLOGIQUE

(30) Priorität: 05.04.2001 DE 10117155; 30.03.2002 DE 10214459
(43) Veröffentlichungstag der Anmeldung: 07.01.2004
(73) Patentinhaber: Eldith GmbH, 98693 Ilmenau (DE)
(72) Erfinder: BERKES, Sebastian, 98693 Ilmenau (DE); IVANOVA, Galina, 98693 Ilmenau (DE); SCHLEGELMILCH, Falk, 98693 Ilmenau (DE); SCHELLHORN, Klaus, 98693 Ilmenau (DE); HUSAR, Peter, 66424 Homburg (DE); HENNING, Günter, 98693 Ilmenau (DE)
(74) Vertreter: Oehmke, Volker
(86) Internationale Anmeldenummer: PCT/DE2002/001320
(87) Internationale Veröffentlichungsnummer: WO 2002/080768

(56) Entgegenhaltungen:
- EP-A- 0 492 635

## Beschreibung

Die Erfindung betrifft eine Anordnung und ein Verfahren zur Erfassung von Signalen biologischen Ursprungs. Die Anwendung dieses Verfahrens und der Anordnung betrifft vorwiegend aber nicht ausschließlich alle Bereiche der Medizin, in denen Biosignale genutzt werden.

Biologische Signale liefern Informationen über die Funktion von Organen innerhalb eines Organismus. Die Auswertung von Biosignalen wird in der Medizin als diagnostisches Hilfsmittel eingesetzt (EKG, EEG, EMG, EOG, ERG, PPT, Atmung, MKG, MEG). Voraussetzung für die Güte der Diagnose ist neben adäquater Signalverarbeitung und Merkmalsextraktion die artefakt- und störungsfreie Signalerfassung.

Hierbei gilt es, folgende Aspekte zu berücksichtigen:
Die Signalpegel liegen nach Wandlung im Bereich von Nano- bis Millivolt innerhalb eines Frequenzbandes von Null bis einige Kilohertz vor,
Im genutzten Frequenzband treten starke Störsignale auf,
Die zu untersuchenden Signalquellen - z.B. elektrophysiologischen Ursprungs - sind hochohmig
Die physikalischen Eigenschaften z. B. der Elektroden verändern sich mit der Zeit (z.B. durch Änderung der Elektrodenübergangsimpedanzen, Elektrodenspannung, Offsetpotentiale, Andruckbedingung, Bewegungsartefakte)

Im Stand der Technik (z.B. EP 492635) sind Signalerfassungssysteme bekannt, die diese Probleme bei sorgfältiger Wahl der Ableitmethodik und entsprechender Verstärkertechnik teilweise beherrschen. Hochwertige kommerzielle Polygraphiesysteme zur Aufzeichnung von Biosignalen unterschiedlichen physiologischen Ursprungs sind sehr kostenintensiv und meist nur für den stationären Einsatz vorgesehen.

Im Folgenden wird als Beispiel zur Erfassung von Signalen biologischen Ursprungs die gegenwärtige Vorgehensweise mittels Verwendung von Elektroden erörtert:
Das biologische Signal wird über Elektroden vom untersuchten Gewebe abgegriffen und über Elektrodenkabel zu einem Differenzverstärker geführt, dessen künstliches Bezugspotential analog aus der Summe aller angeschlossenen Elektroden erzeugt werden kann (Common Average). Diese Messanordnung ist einfach, aber sehr empfindlich gegenüber Störungen. Aus diesem Grund können Messungen - z.B. die des Elektroenzephalogramms (EEG) - nur in störungsarmer Umgebung oder nach aufwändigen Entstörungsmaßnahmen (Faraday-Käfig, Raumschirmung) durchgeführt werden. Der Aufbau dieser Erfassungssysteme ist komplex, da jeder Kanal seine eigene analoge Vorverarbeitungsstufe hat. Dies erhöht Störanfälligkeit, Baugröße und Energiebedarf und erschwert den Parameterabgleich der Kanäle. Der Gleichanteil der Biosignale wird durch eine analoge Hochpassfilterung unterdrückt.

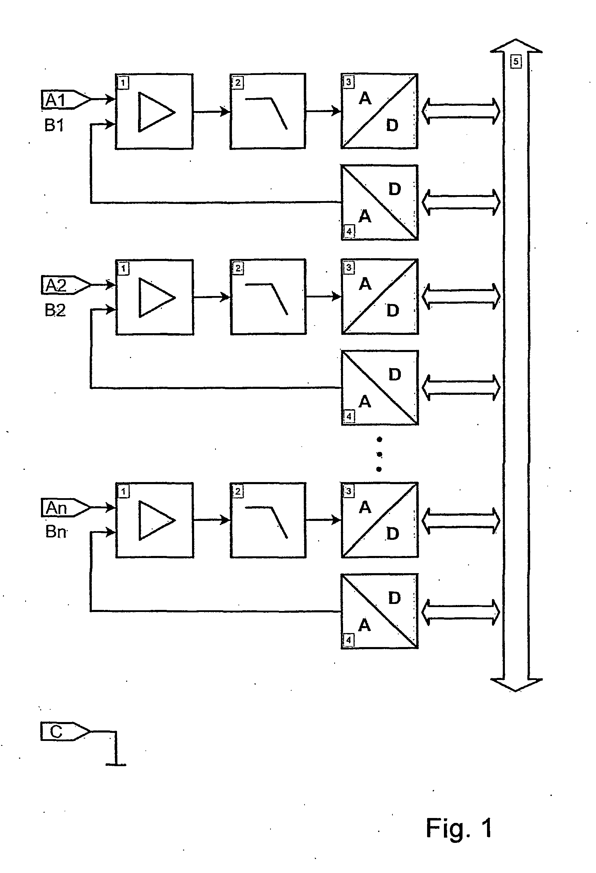

Anspruchsvolle Methoden der Biosignalerfassung und -auswertung benötigen leistungsfähige Biosignalverstärker, welche auch Signalkomponenten im niederfrequenten Bereich bis hin zur Gleichspannung unverzerrt erfassen können. Dies kann realisiert werden, wenn auf eine analoge Hochpassfilterung vollständig verzichtet wird und die gesamte Filterfunktionalität bis auf das Antialiasingfilter auf die digitale Ebene verlagert wird. Alle im vorgestellten System (Figur 1) erzeugten und gemessenen Differenzsignale beziehen sich auf ein gemeinsames Massepotenzial C, welches vom Messobjekt abgeleitet werden kann. Jeder Kanal enthält einen Differenzverstärker 1, ein Antialiasingfilter 2, einen Analog-Digital-Wandler 3, einen Digital-Analog-Wandler 4 und ist von den anderen Kanälen entkoppelt. In allen Kanälen n wird die Differenz zwischen Eingangssignal Aₙ und einem Bezugspotenzial B_{n'} die beide auf das Massepotenzial C bezogen werden, verstärkt, gefiltert und digitalisiert. Das in den Kanalweg eingeschaltete Antialiasingfilter 2 dient der Begrenzung des Frequenzbereiches und somit zur Einhaltung des Abtasttheorems bei der darauffolgenden Quantisierung im Analog-Digital-Wandler 3. Die Daten werden auf einem Daten- und Steuerbus 5 bereitgestellt, entweder im Erfassungssystem selbst oder nach einer Datenübertragung in einem anderen System weiterverarbeitet. Das Bezugspotenzial Bₙ jedes Differenzverstärkers 1 wird aus den Daten des jeweiligen Analog-Digital-Wandlers 3 ermittelt und über einen Digital-Analog-Wandler 4 dem komplementären Eingang zurückgeführt. Auf diese Weise wird einem möglichen Übersteuern des Differenzverstärkers 1 entgegengewirkt, ohne dass die Information über den Gleichanteil verloren geht.

Zur Erfassung des Signals biologischen Ursprungs wird das Differenzsignal zwischen zwei Kanälen, z.B. A₁ und A₂ durch digitale Subtraktion entweder im Erfassungssystem selbst oder nach einer Datenübertragung in ein anderes System gebildet. Auf diese Weise wird es ermöglicht, jeden beliebigen Kanal als Referenzkanal zu deklarieren, um unipolare Ableitungen zu realisieren. Ebenso ist die Definition mehrerer unabhängiger Referenzkanäle, beispielsweise für Biosignale unterschiedlichen Ursprungs, denkbar.
Die eingestellten Verstärkungen für jeden Kanal n sollten für die Erlangung einer hinreichenden Unterdrückung des Einflusses des Gleichtakteingangssignals auf das Ergebnis gleich sein. Die Verstärkung kann so eingestellt werden, dass nahezu alle Biosignale in ihrer Amplitude erfasst werden können, ohne dass es zu einem Informationsverlust durch Übersteuern, Quantisierung oder Systemrauschen kommt.

Die wesentlichen Vorteile dieser Anordnung gegenüber herkömmlichen Lösungen werden im Folgenden genannt:
Es ist keine analoge Hochpassfilterung nötig, somit entfallen Präzisionsbauelemente und deren aufwändiger Parameterabgleich.
Eine Signalerfassung im niederfrequenten Bereich bis hin zur Gleichspannung ist möglich.
Die Datenverarbeitung erfolgt vollständig digital.
Da die Ableitung gegen das Massepotenzial erfolgt, liegen nach der digitalen Differenzbildung unipolare Messdaten vor.
Ausgehend von o.g. unipolaren Messdaten können beliebige Referenzkanäle hardwareunabhängig erzeugt werden.
Eine gleichzeitige Erfassung von Biosignalen unterschiedlichen Ursprungs ist mit verschiedenen Verstärkungsfaktoren und Abtastraten möglich.

Die modulare Hardwarekonzeption der Kanäle und die gemeinsame digitale Schnittstelle ermöglichen eine beliebige Kaskadierbarkeit.
Die Daten werden nicht - wie in konventionellen Systemen - per Zeitmultiplex erfasst, sondern auf Grund der modularen Struktur können sie sowohl simultan als auch völlig unabhängig voneinander abgetastet werden.
Die digitale Schnittstelle ermöglicht eine sehr effiziente galvanische Trennung der Messanordnung von der Auswertetechnik, so dass aufwändige analoge Isolationsverstärker zur Gewährleistung der technischen Sicherheit beim medizinischen Einsatz entfallen, ohne das dadurch die Sicherheit gegenüber dem Messsubjekt (Patient) eingeschränkt wird.
Im Vergleich mit der herkömmlichen Technik zeichnet sich die vorgeschlagene Lösung durch eine geringe Baugröße und einen niedrigen Energiebedarf aus.
Durch geringe Baugröße ist eine Analog-Digital-Wandlung sehr nah an der Signalquelle möglich. Eine Reduktion von Störungen wird somit erreicht, da analoge Signalwege sehr kurz und induktive Störeinkopplungen über Leiterschleifen im analogen Teil der Hardware vermieden werden. Konventionelle Verstärker können induktiv eingekoppelte Störungen nicht vom Nutzsignal trennen, da diese als Differenzeingangsspannung oder -strom vorliegen und mit dem Nutzsignal verstärkt werden.

### Bezugszeichenliste

- 1: Differenzverstärker
- 2: Antialiasingfilter
- 3: Analog-Digital-Wandler
- 4: Digital-Analog-Wandler
- 5: Daten- und Steuerbus

### Abkürzungen

- EKG: Elektrokardiogramm
- EEG: Elektroenzephalogramm
- EMG: Elektromyogramm
- EOG: Elektrookulogramm
- ERG: Elektroretinogramm
- PPT: Photoplethysmographie
- MKG: Magnetkardiogramm
- MEG: Magnetenzephalogramm

## Patentansprüche

1. Verfahren zur Erfassung von Signalen biologischen Ursprungs, wobei
- die aus einer biologischen Quelle stammenden Signale in eine elektrische Größe gewandelt vorliegen, verstärkt und quantisiert werden,
- ein vom Messobjekt abgeleitetes gemeinsames Massepotenzial genutzt wird,
**dadurch gekennzeichnet, dass**
- jeder Kanal sein eigenes digital gesteuertes Bezugspotenzial besitzt.

2. Verfahren nach Anspruch 1, **dadurch gekennzeichnet, dass** die Signale digital auf einen oder mehrere Referenzkanäle bezogen werden können.

3. Verfahren nach einem der Ansprüche 1 oder 2, **dadurch gekennzeichnet, dass** die gleichzeitige Erfassung mehrkanaliger biologischer Signale gleichen und/oder unterschiedlichen Ursprungs möglich ist.

4. Anordnung zur Erfassung von Signalen biologischen Ursprungs, wobei
- die in eine elektrische Größe gewandelten Biosignale mit einem Differenzverstärker 1 verstärkt und mit einem Analog-Digital-Wandler 3 mit vorgeschaltetem Antialisingfilter 2 digitalisiert werden **dadurch gekennzeichnet dass**
- die Bereitstellung des aus den Daten des Analog-Digital-Wandlers 3 gewonnenen Bezugspotentials Bₙ am komplementären Eingang des Differenzverstärkers 1 mit einem Digital-Analog-Wandler 4 realisiert wird.

## Claims

1. A method for detecting signals of biological origin, wherein
- the signals coming from a biological source are present in a form converted into an electrical quantity, are amplified and quantified,
- a common ground potential derived from the object being measured is used,
**characterized in that**
each channel has its own digitally controlled reference potential.

2. The method according to claim 1, **characterized in that** the signals can be digitally referred to one or more reference channels.

3. The method according to any one of claims 1 or 2, **characterized in that** the simultaneous detection of multi-channel biological signals of the same origin and/or of different origins is possible.

4. An assembly for detecting signals of biological origin, wherein
- the biosignals converted into an electrical quantity are amplified by a differential amplifier 1 and are digitized by an analog/digital converter 3 with a preceding anti-aliasing filter 2,
**characterized in that**
- the reference potential Bₙ obtained on the basis of the data of the analog/digital converter 3 is provided to the complementary input of the differential amplifier 1 by a digital/analog converter 4.

## Revendications

1. Procédé de détection de signaux d'origine biologique, dans lequel
- les signaux venant d'une source biologique sont présents sous forme converti en une quantité électrique, et ils sont amplifiés et quantifiés,
- on utilise un potentiel de terre commun dérivé de l'objet à mesurer,
**caractérisé en ce que**
chaque canal présente son propre potentiel de référence numériquement commandé.

2. Procédé selon la revendication 1, **caractérisé en ce que** les signaux peuvent être rapportés numériquement à un ou plusieurs canaux de référence.

3. Procédé selon l'une quelconque des revendications 1 ou 2, **caractérisé en ce que** la détection synchrone de signaux biologiques multicanaux du même origine et/ou d'origines différentes est possible.

4. Ensemble destiné à détecter des signaux d'origine biologique, dans lequel
- les biosignaux convertis en une quantité électrique sont amplifiés par un amplificateur différentiel 1 et sont numérisés par un convertisseur analogique-numérique 3 présentant un filtre anti-alias 2 disposé en aval,
**caractérisé en ce que**
- le potentiel de référence Bₙ obtenu à base des dates du convertisseur analogique-numérique 3 est fourni à l'entrée complémentaire de l'amplificateur différentiel 1 par un convertisseur numérique-analogique 4.
